# EUROPEAN PATENT SPECIFICATION

(11) **EP 2 762 129 B1**
(45) Date of publication and mention of the grant of the patent: **07.08.2019**
(21) Application number: 12836543.4
(22) Date of filing: 28.09.2012
(51) Int. Cl.: C11D 7/26, A61K 8/39

(54) **COSMETIC COMPOSITION AND COSMETIC**
KOSMETISCHE ZUSAMMENSETZUNG UND KOSMETIKUM
COMPOSITION COSMÉTIQUE ET PRODUIT COSMÉTIQUE

(30) Priority: 29.09.2011 JP 2011214366
(43) Date of publication of application: 06.08.2014
(73) Proprietor: The Nisshin OilliO Group, Ltd., Tokyo 104-8285 (JP)
(72) Inventor: MATSUZAWA, Makoto, Yokohama-shi Kanagawa 235-8558 (JP); TAKANASHI, Azusa, Yokohama-shi Kanagawa 235-8558 (JP); TAKAHASHI, Tsuneki, Yokohama-shi Kanagawa 235-8558 (JP); GOTOU, Aki, Yokohama-shi Kanagawa 235-8558 (JP)
(74) Representative: Cabinet Laurent & Charras
(86) International application number: PCT/JP2012/075070
(87) International publication number: WO 2013/047743

(56) References cited:
- EP-A1- 2 468 246
- EP-B1- 0 758 641
- WO-A1-2004/048497
- WO-A1-2004/060077
- JP-A- H08 173 787
- JP-A- 2004 224 726
- JP-A- 2011 099 020
- US-A1- 2006 134 197
- US-A1- 2006 289 834
- US-A1- 2009 196 942
- US-A1- 2009 252 773
- US-A1- 2010 129 303
- US-A1- 2010 215 598

## Description

### TECHNICAL FIELD

The present invention relates to a cosmetic composition containing water and a polyglycerol fatty acid ester obtained by esterifying polyglycerol where the average degree of polymerization is 3 to less than 100 and a fatty acid having 6 to 30 carbon atoms, the cosmetic composition is capable of suppressing generation of odor over time.

### BACKGROUND ART

In various fields such as foods, medicines, cosmetics, and the like, polyglycerol fatty acid esters are widely used as a surfactant such as emulsifiers, solubilizing agents and the like, in terms of safety and function thereof. For example, Patent Document 1 discloses a W/O emulsified cosmetic which includes one or more polyglycerol fatty acid esters, glycerol, oil-base, and water. The W/O emulsified cosmetic exhibits excellent usability, temporal stability, and low-viscosity.

Patent Document 2 discloses a O/W emulsified cosmetic which includes a sucrose fatty acid ester, a polyglycerol fatty acid ester, an oil having a melting point of 60°C or more, a wax, and one or more solid oils selected from hydrocarbons. The O/W emulsified cosmetic provides excellent skin fitness effect and exhibits excellent temporal stability, in particular temporal stability under low temperature condition, in spite of the existence of the solid oil.

Patent Document 3 discloses a cosmetic composition containing a polyglycerol fatty acid ester and a non-ionic surfactant, the composition readily exhibits compatibility with makeup dirt so as to remove the dirt quickly, excellent cleansing power even when skin is wet, excellent usage sensation due to good rinsing property which does not leave an oily feeling on user's skin after washing, highly transparency, and excellent water dispersibility.

On the other hand, as a polymeric compound which is widely used as well as a polyglycerol fatty acid ester, a polyoxyalkylene-modified organopolysiloxane is used.

By blending a polyoxyalkylene-modified organopolysiloxane, each raw material for a cosmetic become compatible each other, and glossiness and smoothness can be improved. Further, the wettability, the foaming properties, the foam controlling properties, the emulsifying properties, the cleaning properties, and the antistatic properties are also improved.

However, a polyoxyalkylene chain within a polyoxyalkylene-modified organopolysiloxane generates aldehydes which cause bad odor by oxidation degradation thereof.

Therefore, there is a problem that, when a polyoxyalkylene-modified organopolysiloxane is used in a cosmetic, bad odor is generated over time.

To solve the problem, Patent Document 4 discloses a polyoxyalkylene-modified organopolysiloxane composition containing a polyoxyalkylene-modified organopolysiloxane and an antioxidant.

Document EP0758641 disclosed merely, at example 69, a composition comprising a fatty acid ester of polyglycerine and citric acid.

Document US2006/289834 is directed to an external composition containing a polyunsaturated fatty acid having at least three double bonds or its salt ester, exhibiting enhanced oxidative stability to suppress the odor derived from polyunsaturated fatty acids. However, polyunsaturated fatty acid is different from polyglycerol fatty acid and it is proved that odor derived from the polyglycerol fatty acid ester cannot be suppressed even when antioxidants such as tocopherol and BHT are used.

Document US2006134197 teaches polyoxyethylene glycol monostearate which is different from polyunsaturated fatty acid and polyglycerol fatty acid ester.

### PRIOR ART DOCUMENTS

### PATENT DOCUMENTS

[Patent Document 1] Japanese Unexamined Patent Application Publication No. H06-128135
[Patent Document 2] Japanese Unexamined Patent Application Publication No. 2009-234971
[Patent Document 3] Japanese Unexamined Patent Application Publication No. 2006-45197
[Patent Document 4] Japanese Unexamined Patent Application Publication No. 2006-176655

### DISCLOSURE OF INVENTION

### PROBLEMS TO BE SOLVED BY THE INVENTION

In the case of a cosmetic and cosmetic composition containing a polyglycerol fatty acid ester and water, there is a problem that bad odor is generated as well as in the case of using a polyoxyalkylene-modified organopolysiloxane.

The present invention has an object to provide a cosmetic composition which contains a polyglycerol fatty acid ester and water and which is capable of suppressing generation of bad odor over time, and to provide a cosmetic containing the cosmetic composition.

### MEANS TO SOLVE THE PROBLEMS

As a result of a variety of extensive and intensive studies to address the problems as described above, the inventors of the present invention have discovered that, by incorporation of a specific compound such as ascorbic acid into a cosmetic composition containing a polyglycerol fatty acid ester and water, the resulting cosmetic composition can suppress the generation of bad odor over time. The present invention has been completed based on these findings.

That is, the present invention relates to a cosmetic composition as defined below:
A cosmetic composition comprising a component (A), a component (B), a component (C), and a component (D), wherein the component (A) is a polyglycerol fatty acid ester, which is an ester of polyglycerol having an average degree of polymerization of 3 to less than 100 with a fatty acid having 6 to 30 carbon atoms, the component (B) is water, the component (C) is at least one water-soluble compound selected from the group consisting of ascorbic acid, salts of ascorbic acid, ascorbic acid derivatives, salts of ascorbic acid derivatives, trehalose, sucrose, citric acid, and salts of citric acid, and the component (D) is at least one oil component selected from the group consisting of natural animal and vegetable fats and oils, semi-synthetic oils, hydrocarbon oils, higher fatty acids, synthetic ester oils, silicone oils, essential oil constituents of animal and vegetable fats and oils, fat-soluble vitamins, phospholipids, higher alcohols, and fluorine-containing oils, wherein the ascorbic acid derivatives is at least one selected from the group consisting of L-ascorbic acid glucoside, L-ascorbic acid-2-phosphate, L-ascorbic acid-3-phosphate, L-ascorbic acid-6-phosphate, L-ascorbic acid-2-polyphosphate, and L-ascorbic acid-2-sulfate, and wherein the amount of the component (A) in the cosmetic composition is 1 to 80% by mass relative to the total amount of the cosmetic composition, the amount of the component (B) in the cosmetic composition is 0.001 to 10% by mass relative to the total amount of the cosmetic composition, the amount of the component (C) in the cosmetic composition is 0.005 to 20% by mass relative to the amount of component (A), the amount of the component (D) in the cosmetic composition is 10 to 90% by mass relative to the total amount of the cosmetic composition.

### EFFECT OF THE INVENTION

According to the present invention, there is provided a cosmetic composition including a polyglycerol fatty acid ester, capable of suppressing generation of bad odor over time, even though the composition contains water, and a cosmetic including the cosmetic composition.

### MODE FOR CARRYING OUT THE INVENTION

The cosmetic composition of a first aspect of the present disclosure contains components (A) to (C) shown below.
Component (A): a polyglycerol fatty acid ester, which is an ester of polyglycerol having an average degree of polymerization of 3 to less than 100 with a fatty acid having 6 to 30 carbon atoms
Component (B): water
Component (C): at least one compound selected from the group consisting of ascorbic acid, salts of ascorbic acid, ascorbic acid derivatives, salts of ascorbic acid derivatives, trehalose, sucrose, citric acid and salts of citric acid

The polyglycerol fatty acid ester as a component (A) is a compound obtainable by esterification of polyglycerol having an average degree of polymerization of 3 to less than 100 with a fatty acid having 6 to 30 carbon atoms.

The fatty acid having 6 to 30 carbon atoms as a raw material of the component (A) may be linear or branched.

The fatty acid may be a saturated fatty acid or an unsaturated fatty acid.

The polyglycerol fatty acid ester obtainable by using a fatty acid having 5 or less carbon atoms tends to cause problems in safety such as skin irritancy.

On the other hand, the polyglycerol fatty acid ester obtainable by using a fatty acid having 31 or more carbon atoms exhibits extremely low solubility in water.

Specific examples of the fatty acids having 6 to 30 carbon atoms include linear saturated fatty acids such as hexanoic acid, octanoic acid (caprylic acid), nonanoic acid, decanoic acid (capric acid), dodecanoic acid (lauric acid), tetradecanoic acid (myristic acid), pentadecanoic acid, hexadecanoic acid (palmitic acid), octadecanoic acid (stearic acid), icosanoic acid, docosanoic acid (behenic acid), and the like; branched saturated fatty acids such as 2-ethylhexanoic acid, 3,5,5-trimethylhexanoic acid, isotridecanoic acid, 2-hexyldecanoic acid, 2-hexyldodecanoic acid, 2-octyldecanoic acid, isostearic acid, 2-octyldodecanoic acid, and the like; and unsaturated fatty acids such as 10-undecenoic acid (undecylenic acid), 9-tetradecenoic acid (myristoleic acid), 2-hexadecenoic acid, 9-hexadecenoic acid, 9-octadecenoic acid (oleic acid), 13-docosene acid, 9,12-octadecadienoic acid (linoleic acid), 6,9,12 -octadecatrienoic acid (linolenic acid), tall acid, eicosapentaenoic acid (EPA), docosahexaenoic acid (DHA), and the like.

As the fatty acids having 6 to 30 carbon atoms as a raw material of the component (A), in terms of reactivity, a fatty acid having 6 to 24 carbon atoms is preferable, a fatty acid having 8 to 22 carbon atoms is more preferable, a fatty acid having 16 to 22 carbon atoms is still more preferable, and a fatty acid having 16 to 20 carbon atoms is particularly preferable.

The polyglycerol as a raw material of the component (A) has an average degree of polymerization of polyglycerol of 3 to less than 100.

As a raw material of the component (A), a polyglycerol having an average degree of polymerization of 3 to 50 is preferable, a polyglycerol having an average degree of polymerization of 3 to 20 is more preferable, and a polyglycerol having an average degree of polymerization of 8 to 12 is still more preferable.

The polyglycerol may be linear, branched or cyclic.

The amount of cyclic compound within a polyglycerol is preferably 30% by mass or less, and more preferably 20% by mass or less.

The amount of cyclic compound can be easily analyzed using LC/MS.

The polyglycerol fatty acid ester as a component (A) is obtainable by esterification of a fatty acid having 6 to 30 carbon atoms with a polyglycerol having an average degree of polymerization of 3 to less than 100.

The polyglycerol fatty acid ester as a component (A) may be obtainable by any synthetic method.

Examples of the production method of a polyglycerol fatty acid ester include an esterification reaction of a polyglycerol with a fatty acid, an ester exchange reaction of a polyglycerol with a fatty acid ester, and an ester exchange reaction of a polyglycerol and an oil or fat.

The polyglycerol fatty acid ester as a component (A) may be a compound in which one mole polyglycerol and one kind of fatty acid form an ester bond, or a compound in which one mole polyglycerol and two or more kinds of fatty acids form ester bonds.

A hydroxyl value of the polyglycerol fatty acid ester as a component (A) is preferably 300 to 700, more preferably 300 to 650, and still more preferably 400 to 630.

When the hydroxyl value is within the range of 300 to 700, water as a component (B) can be blended satisfactory, and a cosmetic composition having excellent handling ability can be obtained.

Further, when the component (A) is blended into a cleansing cosmetic, high level of water-solubilization property is exerted and a cosmetic having high level of cleansing power even when it is used with wet hands can be prepared.

In the present invention, the hydroxyl value can be measured based on The JOCS Standard Methods for the Analysis of Fats, Oils and Related Materials, by determining the number of mg of potassium hydroxide required to neutralize acetic acid necessary for acetylation of free hydroxyl groups contained in 1g of a sample.

As a method of esterification of a fatty acid having 6 to 30 carbon atoms with a polyglycerol having an average degree of polymerization of 3 to less than 100, a conventional esterification method can be mentioned.

Specific examples of the polyglycerol fatty acid ester as a component (A) include a polyglyceryl dioleate having an average degree of polymerization of polyglycerol of 10, a polyglyceryl oleate having an average degree of polymerization of polyglycerol of 6 to 10, a polyglyceryl caprylate having an average degree of polymerization of polyglycerol of 3 to 10, a polyglyceryl laurate having an average degree of polymerization of polyglycerol of 10, a polyglyceryl stearate having an average degree of polymerization of polyglycerol of 10, a polyglyceryl diisostearate having an average degree of polymerization of polyglycerol of 10, and the like.

The cosmetic composition of the first aspect of the present invention may include one kind of polyglycerol fatty acid ester, or two or more kinds of polyglycerol fatty acid esters.

As the component (A), a commercially available compound may be used without any modification.

For example, SALACOS PG-218 (manufactured by Nisshin OilliO Group, Ltd.; active ingredient: polyglycerol dioleate; average degree of polymerization of polyglycerol: 10), SALACOS PG-180 (manufactured by Nisshin OilliO Group, Ltd.; active ingredient: polyglycerol oleate; average degree of polymerization of polyglycerol: 10), and the like can be preferably used.

The amount of the polyglycerol fatty acid ester as a component (A) in the cosmetic composition of the first aspect of the present invention is not particularly limited, as long as the amount is sufficient to exert the desirable properties (e.g., properties as an emulsifier and a solubilizer) in a cosmetic to which the cosmetic composition of the present invention is blended.

For example, in one embodiment of the present invention, the amount of the polyglycerol fatty acid ester as a component (A) in the cosmetic composition of the present invention relative to the total amount of the cosmetic composition is 1 to 80% by mass, more preferably 1 to 60% by mass, and still more preferably 1 to 40% by mass.

In another embodiment of the present invention, the amount of the polyglycerol fatty acid ester as a component (A) in the cosmetic composition of the present invention relative to the total amount of the cosmetic composition is preferably 4 to 50% by mass.

When the amount of the component (A) is within the range of 1 to 80% by mass, the water as a component (B) can be blended satisfactory, and a cosmetic composition and cosmetic can be prepared, in which cleansing power thereof can be maintained even when it is used with wet hands, and rinsing is easily conducted so as not to leave the composition or cosmetic on user's skin, in particular, in the case of a cleansing cosmetic composition or a cleansing cosmetic.

The cosmetic composition of the first aspect of the present invention includes water as a component (B).

The water is not particularly limited, water which is widely used as a raw material of cosmetics can be used, and ion-exchanged water, distilled water, water obtainable from vegetables and fruits, desalted seawater, and the like can be used.

The water obtainable from vegetables and fruits means water which is collected by distillation during preparation of a concentrated liquid of vegetable juice or fruit juice. The water contains a small amount of aroma components and sugars.

The desalted seawater means water which is obtainable by removing salt from seawater or deep ocean water, and which contains a high amount of minerals.

When the cosmetic composition of the present invention is blended in a cleansing cosmetic or a bath cosmetic in which the amount of water is relatively low, it is preferable that the amount of water as a component (B) in the cosmetic composition of the present intention is relatively low.

In one embodiment of the present invention, the amount of water as a component (B) in the cosmetic composition relative to the total amount of the cosmetic composition is 0.001 to 10% by mass.

When the amount of the component (B) is within the range from 0.001 to 10% by mass, low viscosity, excellent handling ability and favorable feeling when it is applied to the skin can be obtained, and water-soluble active ingredients can be blended, and therefore, the cosmetic composition can be used more widely.

The cosmetic composition including a component (A) and a component (B) exhibits low viscosity, excellent handling ability, and favorable feeling when it is applied to the skin, as compared to a composition including just a component (A).

By virtue of the component (B), water-soluble active ingredients can be blended, and therefore, the cosmetic composition can be used more widely.

On the other hand, there is a problem that the composition including a component (A) and a component (B) generates bad odor over time.

The cosmetic composition of the first aspect of the present invention include a specific compound as a component (C), in addition to a polyglycerol fatty acid ester as a component (A) and water as a component (B). Therefore, generation of bad odor from the polyglycerol fatty acid ester as a component (A) over time can be suppressed.

Although the cause of bad odor is not clear, it is presumed that, when a polyglycerol fatty acid ester comes into contact with water, part of the polyglycerol fatty acid is hydrolyzed, thereby generating a substance responsible for bad odor.

As the compound as a component (C), ascorbic acid, salts of ascorbic acid, ascorbic acid derivatives, salts of ascorbic acid derivatives, trehalose, sucrose, citric acid, and salts of citric acid.

These compounds are water-soluble compounds.

The salts of ascorbic acid and salts of citric acid are not particularly limited, as long as they are physiologically acceptable salts and water-soluble salts.

Examples of the water-soluble salts include L-ascorbic acid-2-phosphate magnesium salt, L-ascorbic acid glucoside, sodium citrate, and the like.

As the sodium citrate, trisodium citrate can be mentioned.

The cosmetic composition of the first aspect of the present invention may include one kind of compound as a component (C) or two or more kinds of compounds in combination.

In the present invention, as a component (C), at least one compound selected from the group consisting of ascorbic acid, salts of ascorbic acid, ascorbic acid derivatives, salts of ascorbic acid derivatives, citric acid, salts of citric acid, trehalose and sucrose is preferable. Among these, at least one compound selected from the group consisting of ascorbic acid, L-ascorbic acid-2-phosphate magnesium salt, L-ascorbic acid glucoside, citric acid, sodium citrate, trehalose and sucrose is more preferable.

One compound selected from the group consisting of ascorbic acid, salts of ascorbic acid, ascorbic acid derivatives and salts of ascorbic acid derivatives is still more preferable, and at least one compound selected from the group consisting of ascorbic acid, salts of ascorbic acid, ascorbic acid derivatives and salts of ascorbic acid derivatives is particularly preferable.

Specific examples of water-soluble compounds include L-ascorbic acid as ascorbic acid; and sodium salt, potassium salt, magnesium salt, calcium salt, barium salt, ammonium salt, monoethanolamine salt, diethanolamine salt, triethanolamine salt, monoisopropanolamine salt and triisopropanolamine salt of ascorbic acid as salts of ascorbic acid.

The ascorbic acid derivatives include L-ascorbic acid monoesters such as L-ascorbic acid glucoside, L-ascorbic acid-2-phosphate, L-ascorbic acid-3-phosphate, L-ascorbic acid-6-phosphate, L-ascorbic acid-2-polyphosphate, L-ascorbic acid-2-sulfate.

Examples of the salts of ascorbic acid derivatives include sodium salt, potassium salt, magnesium salt, calcium salt, barium salt, ammonium salt, monoethanolamine salt, diethanolamine salt, triethanolamine salt, monoisopropanolamine salt and triisopropanolamine salt of ascorbic acid derivatives.

Among these, L-ascorbic acid, L-ascorbic acid-2-phosphate magnesium salt, L-ascorbic acid-2-phosphate sodium salt, and L-ascorbic acid glucoside are particularly preferably used.

Examples of the salts of citric acid include sodium citrate, calcium citrate, aluminum citrate, potassium citrate and the like.

As the sodium citrate, trisodium citrate can be mentioned.

The amount of the compound as a component (C) in the cosmetic composition of the first aspect of the present invention is not particularly limited, as long as it is sufficient to suppress generation of bad odor from a polyglycerol fatty acid ester over time. The amount can be appropriately determined taking into consideration of the type or amount of the component (A) and the type of the component (C).

The amount of the compound as a component (C) in the cosmetic composition of the present invention relative to the amount of the component (A) is 0.005 to 20% by mass, more preferably 0.01 to 20% by mass, still more preferably 0.01 to 15% by mass, and particularly preferably 0.01 to 10% by mass.

When the amount of the component (C) is within the range of 0.005 to 20% by mass, the effect can be satisfactorily obtained without precipitation of the component (C).

The cosmetic composition of the first aspect of the present invention includes an oily component as a component (D).

The oily component is not particularly limited, as long as it is oil which is generally used for cosmetics.

The oily component include natural animal and vegetable fats and oils, semi-synthetic oils, hydrocarbon oils, higher fatty acids, synthetic ester oils, silicone oils, essential oil constituents of animal and vegetable fats and oils, fat-soluble vitamins, phospholipids, higher alcohols, fluorine-containing oils.

Among these, natural animal and vegetable fats and oils, semi-synthetic oils, hydrocarbon oils, synthetic ester oils, silicone oils, and essential oil constituents of animal and vegetable fats and oils are preferably used.

Examples of the natural animal and vegetable fats and oils and semi-synthetic oils include avocado oil, linseed oil, almond oil, olive oil, wheat germ oil, sesame oil, rice germ oil, rice bran oil, safflower oil, soybean oil, evening primrose oil, corn oil, rapeseed oil, horse fat, palm oil, palm kernel oil, castor oil, sunflower oil, jojoba oil, macadamia nut oil, coconut oil, hydrogenated coconut oil, peanut oil, lanolin, camellia oil, turtle oil, mink oil, egg yolk oil, persic oil, sasanqua oil, grape seed oil, cottonseed oil, perilla oil, tea seed oil, nutmeg oil, rice bran oil, paulownia oil, Japanese tung oil, and the like.

Among these, olive oil, safflower oil, soybean oil, evening primrose oil, rapeseed oil, palm oil, palm kernel oil, castor oil, jojoba oil, macadamia nut oil, coconut oil, camellia oil, and grape seed oil are preferably used.

Examples of hydrocarbon oils include squalane, squalene, liquid paraffin, isoparaffin, α-olefin oligomers, petrolatum, and the like.

Among these, squalane, liquid paraffin, isoparaffin, and α-olefin oligomer are preferably used.

Examples of the synthetic ester oils include isononyl isononanoate, isodecyl isononanoate, isotridecyl isononanoate, isostearyl myristate, isopropyl myristate, cetyl 2-ethylhexanoate, octyldodecyl myristate, isopropyl palmitate, 2-ethylhexyl palmitate, butyl stearate, hexyl laurate, myristyl myristate, decyl oleate, cetyl octanoate, octyldodecyl oleate, hexyldecyl dimethyloctanoate, cetyl lactate, myristyl lactate, octyldodecyl lactate, tetradecyl lactate, lanolin acetate, isocetyl stearate, isocetyl isostearate, isostearyl isostearate, cholesteryl 12-hydroxystearate, phytosteryl 12-hydroxystearate, phytosteryl oleate, ethylene glycol di-2-ethylhexanoate, dipentaerythritol fatty acid ester, alkyl glycol monoisostearate, neopentyl glycol diethylhexanoate, neopentyl glycol dicaproate, pentaerythritol tetra-2-ethylhexanoate, pentaerythritol tetraisostearate, glyceryl tri-2-ethylhexanoate (triethylhexanoin), glyceryl tri(caprylate/caproate), glyceryl triisostearate, glyceryl tri(caprylate/caproate/myristate/stearate), glyceryl trimyristate, glyceryl tricaprylate, glyceryl tricaproate, glyceryl tri-2-heptylundecanoate, trimethylolpropane triisostearate, trimethylolpropane tri-2-ethylhexanoate, ditrimethylolpropane oligoester (isostearate/sebacate), erythrityl triethylhexanoate, dipentaerythrityl tripolyhydroxystearate, trehalose isostearate esters, dipentaerythrityl pentaisostearate, diglyceryl triisostearate, diglyceryl tetraisostearate, diisostearyl malate, castor oil fatty acid methyl ester, lanolin fatty acid isopropyl ester, oleyl oleate, acetoglyceride, 2-heptylundecyl palmitate, diisobutyl adipate, glyceryl oligomer (adipate /2-ethylhexanoate/stearate), diglyceryl oligoester (2-hexyldecanoate/sebacate), N-lauroyl-L-glutamic acid-2-octyldodecyl ester, di-2-heptylundecyl adipate, hexyl laurate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, 4-methoxycinnamate ester, tetraerythrityl rosinate, glycerol tritetradecanoate, glycerol triisopalmitate, ethyl acetate, butyl acetate, triethyl citrate, glyceryl tri(behenate/isostearate/eicosanedioate), glyceryl (behenate/eicosanedioate), bisethoxydiglycol succinate, neopentylglycol diisononanoate, (polyglyceryl isostearate-2/dimer dilinoleate) copolymer, hydrogenated castor oil dimer dilinoleate, di (caprylic acid/capric acid) propanediol ester, propanediol diisostearate, polyglyceryl-6 octacaprylate, bisethoxydiglycol cyclohexane-1,4-dicarboxylate, glyceryl di-p-methoxycinnamate mono-iso-octylate.

Among these, isononyl isononanoate, cetyl ethylhexanoate, glyceryl (behenate/eicosanedioate), and glyceryl tri-2-ethylhexanoate (triethylhexanoin) are preferably used.

Examples of the silicon oil include chain polysiloxanes such as dimethyl polysiloxane, methylphenyl polysiloxane and methylhydrogen polysiloxane; cyclic polysiloxanes such as octamethylcyclotetrasiloxane, decamethylcyclopentasiloxane, dodecamethylcyclohexasiloxane and tetrahydrotetramethylcyclotetrasiloxane; polyoxyethylene polyalkyl siloxane, higher alkoxy-modified silicones such as stearoxy silicone, alkyl modified silicones, higher fatty acid ester-modified silicones, and the like.

Examples of the higher fatty acid include lauric acid, myristic acid, palmitic acid, stearic acid, behenic acid, oleic acid, undecylenic acid, tall oil acid, isostearic acid, linoleic acid, linolenic acid, eicosapentaenoic acid (EPA) and docosahexaenoic acid (DHA).

Examples of the higher alcohol include linear alcohols such as lauryl alcohol, cetyl alcohol, stearyl alcohol, behenyl alcohol, myristyl alcohol, oleyl alcohol, and cetostearyl alcohol; and branched alcohols such as monostearyl glycerin ether (batyl alcohol), 2-decyl tetra decynol, lanolin alcohol, cholesterol, phytosterol, hexyl dodecanol, isostearyl alcohol and octyl dodecanol.

Examples of the phospholipid include lecithins and hydrogenated lecithins such as soybean phospholipids, hydrogenated soybean phospholipids, rapeseed phospholipids, hydrogenated rapeseed phospholipids, egg yolk phospholipids and hydrogenated egg yolk phospholipids.

Among these, hydrogenated soybean phospholipids, hydrogenated rapeseed phospholipids, and hydrogenated egg yolk phospholipids are preferably used.

Examples of the fluorine-containing oil include perfluorodecaline, perfluoroadamantane, perfluorobutyltetrahydrofuran, perfluorooctane, perfluorononane, perfluoropentane, perfluorodecane, perfluorododecane, and the like.

Examples of fat-soluble vitamin include tocopherol and derivatives thereof, retinol and derivatives thereof and the like.

Among these, as the component (D), isononyl isononanoate, cetyl ethylhexanoate, glyceryl (behenate/eicosanedioate), liquid paraffin, triethylhexanoin, and hydrogenated lecithins are preferably used.

The cosmetic composition of the first aspect of the present invention may include one kind of oily component as a component (D), or two or more kinds of oily components.

As the component (D), a commercially available compound may be used without any modification.

The amount of the oily component as a component (D) in the cosmetic composition of the first aspect of the present invention relative to the total amount of the cosmetic composition is 10 to 90% by mass, more preferably 20 to 85% by mass, still more preferably 25 to 80% by mass, and particularly preferably 55 to 80% by mass.

When the amount of the component (D) is within the range of 10 to 90% by mass, a cosmetic composition has favorable skin blendability. In particular, in the case where the cosmetic composition is used in a cleansing cosmetic composition or a cleansing cosmetic, the composition or cosmetic exhibits favorable compatibility with makeup and excellent cleansing power.

The cosmetic composition of the first aspect of the preset invention may further include a non-ionic surfactant as a component (E), as well as the components (A) to (C). For example, the cosmetic composition of the first aspect of the present invention may include a non-ionic surfactant as a component (E) in the amount of 0.0001 to 60% by mass relative to the total amount of the cosmetic composition. When the cosmetic composition is blended in a cosmetic such as a cleansing cosmetic which is required to have a high level of cleaning power, in an embodiment, the amount of a non-ionic surfactant as a component (E) relative to the total amount of the cosmetic composition is preferably 1 to 40% by mass, and more preferably 2 to 30% by mass.

In an another embodiment, the amount of a non-ionic surfactant as a component (E) relative to the total amount of the cosmetic composition is preferably 0.7 to 6% by mass.

When the component (E) is used within the range of 1 to 40% by mass with component (A) in combination, a cosmetic composition can be prepared, which has excellent stability in the case where a component (D) is blended in the cosmetic composition.

Further, when the cosmetic composition is used for a cleansing cosmetic composition or a cleansing cosmetic, a cosmetic composition or a cleansing cosmetic can be prepared, which has favorable compatibility with makeup and excellent cleansing power.

When an oily component as a component (D) and a non-ionic surfactant as a component (E) are used in combination, the mix ratio represented by mass ratio of the oily component as a component (D) and the non-ionic surfactant as a component (E) is preferably 10:0.1 to 0.1:10.

In the case where the mix ratio of the oily component as a component (D) and the non-ionic surfactant as a component (E) is within the aforementioned range, the function of the polyglycerol fatty acid ester as a component (A) can be maintained without loss of the function, even when the cosmetic composition of the present invention is blended in a cleansing cosmetic or a bath cosmetic in which the amount of water is relatively low.

As the non-ionic surfactant as a component (E), surfactants having no group to be ionized as a hydrophilic group can be mentioned, and specific example thereof include surfactants having a polyoxyalkylene group such as polyoxyethylene alkyl ethers, glycerin fatty acid esters, polyglycerol fatty acid esters, polyalkylene glycol fatty acid esters, sorbitan fatty acid esters, sucrose fatty acid esters, pentaerythritol fatty acid esters, fatty acid alkanolamides, ethers composed of a mono or polyhydric alcohol and a polyoxyalkylene glycol, ethers composed of a polyoxyalkylene and a sugar, condensation products of a polyoxyalkylene glycol and an aliphatic amine, alkyl or polyalkenyl glycosides, and the like.

The polyglycerol fatty acid ester as a component (A) is not regarded as a non-ionic surfactant as a component (E).

Examples of the non-ionic surfactant include polyoxyethylene (POE) sorbitan fatty acid esters such as POE sorbitan monooleate, POE sorbitan monostearate, POE sorbitan monooleate and POE sorbitan tetraoleate; POE sorbitol fatty acid esters such as POE sorbitol monolaurate, POE sorbitol monooleate, POE sorbitol pentaoleate and POE sorbitol monostearate; POE glycerol fatty acid esters such as POE glycerol monostearate, POE glycerol monoisostearate and POE glycerol triisostearate; POE fatty acid esters such as POE monooleate, POE distearate, POE monodioleate and ethylene glycol distearate; POE alkyl ethers such as POE lauryl ether, POE oleyl ether, POE stearyl ether, POE behenyl ether, POE 2-octyldodecyl ether and POE cholestanol ether; Pluronic-type surfactants such as Pluronic; POE-POP alkyl ethers such as POE-POP cetyl ether, POE-POP 2-decyltetradecyl ether, POE-POP monobutyl ether, POE-POP hydrogenated lanolin and POE-POP glycerol ether; tetra-POE-tetra-POP ethylenediamine condensates such as Tetronic; POE castor oil/ hydrogenated castor oil derivatives such as POE castor oil, POE hydrogenated castor oil, POE hydrogenated castor oil monoisostearate, POE hydrogenated castor oil triisostearate, POE hydrogenated castor oil monopyroglutamate monoisostearate diester and POE hydrogenated castor oil maleate; POE beeswax lanolin derivatives such as POE sorbitol beeswax; alkanolamides such as coconut oil fatty acid diethanolamide (Cocamide DEA), lauric acid monoethanolamide and fatty acid isopropanolamides; POE propylene glycol fatty acid esters; POE alkylamines; POE fatty acid amides; sucrose fatty acid esters; POE nonylphenyl formaldehyde condensates; alkylethoxydimethylamine oxides; trioleyl phosphoric acid; modified silicones such as methyl polysiloxane-cetylmethyl polysiloxane-poly(oxyethylene-oxypropylene) methyl polysiloxane copolymer; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate and diglycerol sorbitan tetra-2-ethylhexylate; propylene glycol fatty acid esters such as propylene glycol monostearate; glycerin fatty acid esters such as hydrogenated castor oil derivatives, glycerin alkyl ethers, decyl glucoside, cottonseed oil fatty acid monoglycerides, erucic acid monoglycerides, glyceryl sesqui oleate, glyceryl monostearate, pyroglutamic acid glyceryl α,α-oleate, glyceryl monostearate and glyceryl monooleate; diglycerin fatty acid esters such as diglyceryl monoisostearate, diglyceryl diisostearate and diglyceryl condensed ricinoleate; and polyglycerin fatty acid esters where the average degree of polyglycerol is 2 or less, such as polyglyceryl sesquicaprylate, polyglyceryl dicaprylate, polyglyceryl monolaurate, polyglyceryl monostearate, polyglyceryl monooleate, polyglyceryl distearate and polyglyceryl dioleate.

Here, "POE" means a polyoxyethylene group, and "POP" means a polyoxypropylene group.

As the non-ionic surfactant as a component (E), a polyhydric alcohol fatty acid ester obtaineable by esterification of a fatty acid having 6 to 22 carbon atoms with a polyhydric alcohol having 2 to 4 hydroxyl groups can be mentioned. It is preferable that a polyhydric alcohol fatty acid ester is a mixture containing a polyhydric alcohol fatty acid monoester (hereafter, sometimes referred to as "monoester") and a polyhydric alcohol fatty acid diester (hereafter, sometimes referred to as "diester"), wherein the sum of monoesters and diesters is at least 50% by mass relative to the total amount of the polyhydric alcohol fatty acid ester, and the mass ratio of monoesters and diesters represented by [mass of monoesters] / [mass of diesters] is 12 or less. It is more preferable that the mass ratio is 4 or less, and it is still more preferable that the mass ratio is 2.3 to 1.4.

The fatty acid having 6 to 22 carbon atoms, which is used as a raw material for a polyhydric alcohol fatty acid ester may be linear or branched. The fatty acid may be a saturated fatty acid or an unsaturated fatty acid.

Specific examples of the fatty acid having 6 to 22 carbon atoms include linear saturated fatty acids such as hexanoic acid, octanoic acid (caprylic acid), nonanoic acid, decanoic acid (capric acid), dodecanoic acid (lauric acid), tetradecanoic acid (myristic acid), pentadecanoic acid, hexadecanoic acid (palmitic acid), octadecanoic acid (stearic acid), icosane acid, and docosanoic acid (behenic acid); branched saturated fatty acids such as 2-ethylhexanoic acid, 3,5,5-trimethylhexanoic acid, isotridecanoic acid, 2-hexyldecanoic acid, 2-hexyldodecanoic acid, 2-octyldecanoic acid, isostearic acid, and 2-octyldodecanoic acid; and unsaturated fatty acids such as 2-octyldodecanoic acid, 1 0-undecenoic acid (undecylenic acid), 9-tetradecenoic acid (myristoleic acid), 2-hexadecenoic acid, 9-hexadecenoic acid, 9-octadecenoic acid (oleic acid), 13-docosene acid, 9, 12-octadecadienoic acid (linoleic acid), 6,9,12-octadecatrienoic acid (linolenic acid), tall acid, eicosapentaenoic acid (EPA), and docosahexaenoic acid (DHA).

Examples of the polyhydric alcohol having 2 to 4 hydroxyl groups, which is used as a raw material for a polyhydric alcohol fatty acid ester, include propylene glycol, glycerol, di-glycerol, 1,3-butylene glycol, isoprene glycol, dipropylene glycol, polyethylene glycol, pentaerythritol, neopentyl glycol, sorbitan, and the like.

Among these, it is preferable that one or two or more alcohols selected from the group consisting of glycerol, di-glycerol, sorbitan, and pentaerythritol.

The polyhydric alcohol fatty acid ester as a component (E) is obtainable by esterification of a fatty acid having 6 to 22 carbon atoms with a polyhydric alcohol having 2 to 4 hydroxyl groups.

The polyhydric alcohol fatty acid ester as a component (E) may be obtainable by any synthetic method.

As the production method of a polyhydric alcohol fatty acid ester, an esterification reaction of a polyhydric alcohol and a fatty acid, an ester exchange reaction of a polyhydric alcohol and a fatty acid ester, and an ester exchange reaction of a polyhydric alcohol and an oil or fat can be mentioned.

A polyhydric alcohol fatty acid ester may be obtained through distillation purification and a decolonization treatment as needed, and may be obtained by blending two or more kinds of polyhydric alcohol fatty acid esters.

The polyhydric alcohol fatty acid ester as a component (E) may be a polyhydric alcohol fatty acid ester in which one kind of fatty acid and one mole of polyhydric alcohol form an ester bond, or a polyhydric alcohol fatty acid ester in which two or more kinds of fatty acids and one mole of polyhydric alcohol form ester bonds.

When the cosmetic composition of the first aspect of the present invention is blended in a cosmetic such as a cleansing cosmetic which is required to have a high level of cleaning power, the sum of monoesters and diesters in a polyhydric alcohol fatty acid ester as a component (E) is preferably at least 50% by mass, and more preferably at least 60% by mass, relative to the total amount of the polyhydric alcohol fatty acid ester.

When the sum of monoesters and diesters is at least 50% by mass, the solubilization ratio of water can be satisfactorily enhanced.

In this case, the mass ratio of monoesters and diesters represented by [mass of monoesters] / [mass of diesters] is preferably 12 or less, more preferably 2.3 to 12, still more preferably 4 or less, particularly preferably 2.3 to 0.4, and most preferably 1.5 to 0.4.

When the mass ratio of monoesters and diesters is 12 or less, microemulsion can be reliably formed in the presence of water.

The cosmetic composition of the first aspect of the present invention may include one kind of non-ionic surfactant as a component (E) or two or more kinds of non-ionic surfactants.

As the component (E), a commercially available non-ionic surfactant may be used without any modification.

For example, polyglyceryl sesquicaprylate (average degree of polymerization of polyglycerol: 2, sum of monoesters and diesters: 68%, mass ratio of monoesters and diesters: 1) and polyglyceryl oelate (average degree of polymerization of polyglycerol: 2, sum of monoesters and diesters: 100%, mass ratio of monoesters and diesters: 11) can be preferably used.

For the purpose of functional improvement, providing nutrients to the skin, keeping quality from deteriorating, the cosmetic composition of the first aspect of the present invention may further appropriately contain various components which have been commonly used in cosmetic compositions and do not fall in the definitions of components (A) to (E), if necessary, within the range where the effect of the present invention is not impaired.

Specific examples thereof include water-soluble natural polymers, water-soluble semi-synthetic polymers, water-soluble synthetic polymers, water-soluble inorganic polymers, ultraviolet absorbing agents, sequestering agents, lower alcohols, polyhydric alcohols, monosaccharides, oligosaccharides, polysaccharides, amino acids, organic amines, synthetic resin emulsions, preservatives, pH adjusting agents, vitamins, plant extracts, antioxidants, antioxidant aids, perfumes, and the like.

These components may be used alone or in a combination of two or more kinds thereof.

Examples of the water-soluble natural polymers include plant-based polymers such as gum arabic, tragacanth gum, galactan, guar gum, carob gum, karaya gum, carrageenan, pectin, quince seed (marmelo), algae colloid (brown algae extract), and starch (rice, corn, potato, wheat); microorganism-based polymers such as dextran, succinoglucan, and bull run; and animal polymers such as collagen, casein, albumin, and gelatin.

Examples of the water-soluble semi-synthetic polymers include starch-based polymers such as a carboxymethyl starch and a methyl hydroxypropyl starch; cellulose-based polymers such as methyl cellulose, nitro cellulose, methyl hydroxypropyl cellulose, sodium cellulose sulfate, hydroxypropyl cellulose, carboxymethyl cellulose, sodium carboxymethyl cellulose, crystalline cellulose, and cellulose powder; and alginic acid-based polymers such as sodium alginate, and propylene glycol alginate.

Examples of the water-soluble synthetic polymers include vinyl-based polymers such as polyvinyl alcohol, polyvinyl methyl ether, polyvinyl pyrrolidone, and carboxy vinyl polymer (Carbopol); polyoxyethylene polymers such as polyethylene glycol 20,000, polyethylene glycol 40,000, and polyethylene glycol 60,000; copolymerized polymers such as polyoxyethylene polyoxypropylene copolymer; and acrylate-based polymers such as sodium polyacrylate, polyethyl acrylate, and polyacrylamide; polyethylene imine; and cation polymer.

Examples of the water-soluble inorganic polymers include bentonite, magnesium aluminum silicate (Veegum), Laponite, hectorite, and silicic acid anhydride.

Examples of the ultraviolet absorbers include benzoic acid-type ultraviolet absorbers such as p-aminobenzoic acid (hereinafter, abbreviated as "PABA"), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester; anthranilic acid-type ultraviolet absorbers such as homomenthyl-N-acetyl anthranilate; salicylic acid-type ultraviolet absorbers such as amyl salicylate, menthyl salicylate, homomenthyl salicylate, octyl salicylate, phenyl salicylate, benzyl salicylate, and p-isopropanol phenyl salicylate; cinnamic acid-type ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, and glyceryl mono-2-ethylhexanoyl-diparamethoxycinnamate; benzophenone-type ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone; 3-(4'-methylbenzylidene)-d,l-camphor; 3-benzylidene-d,l-camphor; urocanic acid; ethyl urocanate; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one; 2,4,6-trianilino-p-(carbo-2'-ethylhexyl-1'-oxy)-1,3,5-triazine; and 4-tert-butyl-4'-methoxydibenzoylmethane. These ultraviolet absorbers may be used alone or in a combination of two or more kinds thereof.

Among ultraviolet absorbers, diethylamino hydroxybenzoyl hexyl benzoate (for example, product name: Uvinul A+, manufactured by BASF), ethylhexyl triazine (for example, product name Uvinul T-150, manufactured by BASF), t-butyl methoxy dibenzoylmethane (for example, product name: Parsol 1789, manufactured by DSM), bis-ethylhexyloxyphenol methoxyphenyl triazine (for example, product name: Tinosorb S, manufactured by Ciba), diethylhexyl butamidotriazone (for example, product name: Uvasorb HEB 3V, manufactured by Sigma), and oxybenzon-3 (for example, product name Uvinul M-40, manufactured by BASF) are known to have low solubility in silicone oil. When silicone oil is compatible with the oil solution in which ultraviolet absorber has been dissolved, the solubility of ultraviolet absorber is deteriorated, and deposition of ultraviolet absorber may occur. In the cosmetic composition of the present invention, even when silicone oil is incompatible with the oil solution in which ultraviolet absorber has been dissolved, the emulsified state can be stably maintained, and hence, deposition of ultraviolet absorber can be suppressed.

Examples of the sequestering agents include disodium edetate, edetic acid salts, and hydroxyethane diphosphonate. These sequestering agents may be used alone or in a combination of two or more kinds thereof.

Examples of the lower alcohols include methanol, ethanol, propanol, isopropanol, isobutyl alcohol and t-butyl alcohol.

Examples of the polyhydric alcohols include the same polyhydric alcohol having 2 to 4 hydroxy group as those exemplified above as a raw material of the fatty acid ester. Among these, propyrene glycol and glycerol are preferable.

Examples of the monosaccharides include trioses such as D-glyceryl aldehyde, and dihydroxyacetone; tetroses such as D-erythrose, D-erythrulose, and D-threose; pentoses such as L-arabinose, D-xylose, L-lyxose, D-arabinose, D-ribose, D-ribulose, D-xylulose, and L-xylulose; hexoses such as D-glucose, D-talose, D-psicose, D-galactose, D-fructose, L-galactose, L-mannose, and D-tagatose; heptoses such as aldoheptose, heptulose; octoses; deoxy sugars such as 2-deoxy-D-ribose, 6-deoxy-L-galactose, and 6-deoxy-L-mannose; amino sugars such as D-glucosamine, D-galactosamine, sialic acid, aminouronic acid, and muramic acid; and uronic acids such as D-glucuronic acid, D-mannuronic acid, L-guluronic acid, D-galacturonic acid, and L-iduronic acid.

Examples of the oligosaccharides include gentianose, umbelliferose, lactose, planteose, isolychnoses, raffinose, lychnoses, umbilicin, stachyose, and verbascoses.

Examples of the polysaccharides include cellulose, chondroitin sulfate, dextrin, glucomannan, chitin, galactan, dermatan sulfate, glycogen, gum arabic, heparan sulfate, tragacanth gum, keratan sulfate, chondroitin, mucoitin sulfuric acid, guar gum, dextran, kerato sulfate, locust bean gum, succinoglucan, and charonic acid.

Examples of the amino acids include neutral amino acids such as threonine and cysteine; and basic amino acids such as hydroxylysine.

Examples of amino acid derivatives include acyl sarcosine sodium salt (sodium lauroyl sarcosinate), acyl glutamate salt, acyl β-alanine sodium salt, and glutathione.

Examples of the amino acids include monoethanolamine, diethanolamine, triethanolamine, morpholine, triisopropanolamine, 2-amino-2-methyl-1,3-propanediol, and 2-amino-2-methyl-1-propanol.

Examples of the synthetic resin emulsions include alkyl acrylate copolymer emulsion, alkyl methacrylate polymer emulsion, alkyl acrylate copolymer emulsion, alkyl methacrylate copolymer emulsion, acrylic acid/alkyl acrylate copolymer emulsion, methacrylic acid/alkyl methacrylate copolymer emulsion, alkyl acrylate/styrene copolymer emulsion, alkyl methacrylate/styrene copolymer emulsion, vinyl acetate polymer emulsion, polyvinyl acetate emulsion, vinyl acetate-containing copolymer emulsion, vinyl pyrrolidone/styrene copolymer emulsion, and silicone-containing copolymer emulsion. These synthetic resin emulsions may be used alone or in a combination of two or more kinds thereof.

Examples of the preservatives include methylparaben, ethylparaben and phenoxyethanol. These preservatives may be used alone or in a combination of two or more kinds thereof.

Examples of the pH adjusting agents include edetic acid, disodium edetate, sodium hydroxide, potassium hydroxide, and triethanolamine. These pH adjusting agents may be used alone or in a combination of two or more kinds thereof.

Examples of the vitamins include vitamin A, vitamin B1, vitamin B2, vitamin B6, vitamin E, and vitamin K, and derivatives thereof, pantothenic acid, derivatives thereof, and biotin.

Examples of the plant extracts include *Aloe vera,* witch hazel, *Hamamelis,* cucumber, lemon, lavender and rose extracts.

Examples of the antioxidants include oil-soluble vitamin C derivatives, tocopherols, derivatives of tocopherols, and salts thereof; dibutylhydroxytoluene; butylhydroxyanisole; and gallic acid ester. These antioxidants may be used alone or in a combination of two or more kinds thereof.

Examples of the antioxidant aids include phosphoric acid, citric acid, maleic acid, malonic acid, succinic acid, fumaric acid, cephalin, hexametaphosphate, phytic acid, and ethylenediaminetetraacetic acid.

The cosmetic composition of the first aspect of the present invention is used as a raw material of a cosmetic, and is also used as a raw material of the quasi-drugs, pharmaceuticals, and detergents.

The cosmetic composition of the first aspect of the present invention is preferably used as a raw material of a cosmetic such as a cleansing cosmetic or a bath cosmetic in which the amount of water is relatively low.

When the cosmetic composition of the first aspect of the present invention is used as a raw material of a cosmetic, the amount of the cosmetic composition of a first aspect of the present invention relative to the total amount of the cosmetic is preferably 10 to 90% by mass.

The cosmetic composition of the first aspect of the present invention may be used as a composite raw material in which ingredients have been mixed in advance, or may be added as one ingredient of the composite raw materials to the cosmetic.

An embodiment of the present invention is a use of the cosmetic composition of a first aspect of the present invention in the manufacture of a cosmetic.

A method of manufacturing the cosmetic composition of the first aspect of the present invention preferably includes a step in which the component (B) and the component (C) are mixed, and the component (A) is added thereto to prepare a cosmetic composition.

When the cosmetic composition includes the component (D) and the component (E), it is preferable that the method includes a step in which the component (B) and the component (C) are mixed, and the obtained mixture is added to a mixture in which the component (A), the component (D), and the component (E) have been mixed in advance to prepare a cosmetic composition.

There is no particular limitation of the formulation of the cosmetic including the cosmetic composition of the first aspect of the present invention, and the cosmetic composition is preferably used in cleansing cosmetic, milky lotion, cream, serum, lotion, ointment, and pack.

For the purpose of functional improvement, providing nutrients to the skin, keeping quality from deteriorating, the cosmetic including the cosmetic composition of a first aspect of the present invention may further appropriately contain various components commonly used in cosmetics, if necessary, within the range where the effect of the present invention is not impaired.

Specific examples thereof include a surfactant such as a non-ionic surfactant, an anionic surfactant, a cationic surfactant, and an amphoteric surfactant; a powder component such as an inorganic pigment, an organic pigment, iron oxide, and talc; a water-soluble natural polymer, a water-soluble semi-synthetic polymer, a water-soluble synthetic polymer, a water-soluble inorganic polymer, an ultraviolet absorber; a sequestering agent; a lower alcohol, a monosaccharide, an oligosaccharide, a polysaccharide, an amino acid, an organic amine, a synthetic resin emulsion, a salt, a preservative, a pH adjusting agent, a vitamin, a plant extract, an antioxidant, an antioxidant aid, and a perfume. These components may be the same as those described above, and may be used alone or in a combination of two or more kinds thereof.

The cosmetic including the cosmetic composition of the first aspect of the present invention can be produced in the same manner as in the method of producing a conventional cosmetic.

Specifically, a method including a step in which the cosmetic composition of a first aspect of the present invention and the components described above as components which have been commonly used in cosmetic compositions are mixed to obtain a cosmetic can be mentioned.

Further, the obtained cosmetic may be formed in a desirable formulation by a conventional method.

Alternatively, a cosmetics may be obtained by preparing a cosmetic containing one or more components selected from the components (A) to (C) (if necessary, further containing the component (D) and/or the component (E)) describe above in relation to the cosmetic composition of the present invention, and then adding the other components or a cosmetic raw material containing the other components thereto to complete the cosmetic.

The cosmetic of the second aspect of the present invention is a cosmetic which includes one or more components (A) to (C) described above in relation to the cosmetic composition of the first aspect of the present invention (if necessary, further includes the component (D) and/or the component (E). The cosmetic of the second aspect of the present invention may be prepared by adding other raw materials to the cosmetic composition of the first aspect of the present invention, or may be prepared by separately adding each component (A) to (C) together with other raw materials.

The method of deodorization of a water-containing cosmetic is characterized by adding a component (C) to a water-containing cosmetic which contains a component (A) and a component (B). When the appropriate amount of one or more compounds as a component (C) to a water-containing cosmetic which contains a polyglycerol fatty acid ester as a component (A) and water as a component (B), generation of bad odor from the water-containing cosmetic after being stored can be suppressed.

The method of deodorization of a water-containing cosmetic may further contain adding a component (D) to a water-containing cosmetic which contains a component (A) and a component (B).

The method of deodorization of a water-containing cosmetic may further contain adding a component (E) to a water-containing cosmetic which contains a component (A) and a component (B).

When a component (C), component (D), and component (E) are added to a water-containing cosmetic which contains a component (A) and a component (B), it is preferable that the method contains adding a component (D) and a component (E) to a component (A) to obtain a mixture, and subsequently adding a component (B) and a component (C), which have been mixed in advance, to the obtained mixture containing the components (A), (D) and (E).

A cosmetic composition of the first aspect of the present invention preferably includes a component (A), a component (B) and a component (C), wherein the component (A) is a polyglycerol fatty acid ester, which has a hydroxy value of 400 to 630 and is an ester of polyglycerol having an average degree of polymerization of 8 to 12 with a fatty acid having 16 to 20 carbon atoms, the component (B) is water, and the component (C) is at least one compound selected from the group consisting of ascorbic acid, L-ascorbic acid-2-phosphate magnesium salt, L-ascorbic acid glucoside, citric acid, sodium citrate, trehalose and sucrose.

A cosmetic composition of the first aspect of the present invention preferably includes a component (A), a component (B) and a component (C), wherein the component (A) is at least one polyglycerol fatty acid ester selected from the group consisting of a polyglyceryl dioleate having an average degree of polymerization of polyglycerol of 10, a polyglyceryl oleate having an average degree of polymerization of polyglycerol of 6 to 10, a polyglyceryl caprylate having an average degree of polymerization of polyglycerol of 3 to 10, a polyglyceryl laurate having an average degree of polymerization of polyglycerol of 10, a polyglyceryl stearate having an average degree of polymerization of polyglycerol of 10 and a polyglyceryl diisostearate having an average degree of polymerization of polyglycerol of 10, the component (B) is water, and the component (C) is at least one compound selected from the group consisting of ascorbic acid, L-ascorbic acid-2-phosphate magnesium salt, L-ascorbic acid glucoside, citric acid, sodium citrate, trehalose and sucrose.

A cosmetic composition of the first aspect of the present invention preferably includes a component (A), a component (B) and a component (C), wherein the component (A) is a polyglyceryl dioleate having an average degree of polymerization of polyglycerol of 10 and having a hydroxyl value of 300 to 700 or a polyglyceryl oleate having an average degree of polymerization of polyglycerol of 10 and having a hydroxyl value of 300 to 700, the component (B) is water, and the component (C) is at least one compound selected from the group consisting of ascorbic acid, L-ascorbic acid-2-phosphate magnesium salt, L-ascorbic acid glucoside, citric acid, sodium citrate, trehalose and sucrose.

A cosmetic composition of the first aspect of the present invention preferably includes a component (A), a component (B), a component (C), a component (D) and a component (E), wherein the component (A) is a polyglyceryl dioleate having an average degree of polymerization of polyglycerol of 10 and having a hydroxyl value of 300 to 700 or a polyglyceryl oleate having an average degree of polymerization of polyglycerol of 10 and having a hydroxyl value of 300 to 700, the component (B) is water, the component (C) is at least one compound selected from the group consisting of ascorbic acid, L-ascorbic acid-2-phosphate magnesium salt, L-ascorbic acid glucoside, citric acid, sodium citrate, trehalose and sucrose, the component (D) is at least one compound selected from the group consisting of isononyl isononanoate, cetyl ethylhexanoate, glyceryl (behenate/eicosanedioate), liquid paraffin, triethylhexanoin and hydrogenated lecithins, and the component (E) is a polyhydric alcohol fatty acid ester, which is an ester of a fatty acid having 6 to 22 carbon atoms with a polyhydric alcohol having 2 to 4 hydroxyl groups, wherein the sum of monoesters and diesters is at least 50% by mass relative to the total amount of the polyhydric alcohol fatty acid ester, and the mass ratio of monoesters and diesters represented by [mass of monoesters] / [mass of diesters] is 12 or less.

A cosmetic composition of the first aspect of the present invention preferably includes a component (A), a component (B), a component (C), a component (D) and a component (E), wherein the component (A) is a polyglyceryl dioleate having an average degree of polymerization of polyglycerol of 10 and having a hydroxyl value of 300 to 700 or a polyglyceryl oleate having an average degree of polymerization of polyglycerol of 10 and having a hydroxyl value of 300 to 700, the component (B) is water, the component (C) is at least one compound selected from the group consisting of ascorbic acid, L-ascorbic acid-2-phosphate magnesium salt, L-ascorbic acid glucoside, citric acid, sodium citrate, trehalose and sucrose, the component (D) is at least one compound selected from the group consisting of isononyl isononanoate, cetyl ethylhexanoate, glyceryl (behenate/eicosanedioate), liquid paraffin, triethylhexanoin and hydrogenated lecithins, and the component (E) is polyglyceryl sesquicaprylate having an average degree of polymerization of polyglycerol of 2 or polyglyceryl oleate having an average degree of polymerization of polyglycerol of 2.

### EXAMPLES

The present invention will now be described in more detail with reference to Examples of the present invention and Comparative Examples. However, it is apparent that the present invention is not limited to the following Examples.

### [Examples 1 and 2, Comparative Examples 1 to 5]

### (Production method)

All components (except for water, ascorbic acid and L-ascorbic acid-2-phosphate magnesium salt) were weighed in accordance with the composition shown in Table 1, added to a beaker and mixed, and heated to 80°C to be dissolved, followed by gradually cooling while stirring. After cooling, to the obtained mixture, a mixture in which water, ascorbic acid and L-ascorbic acid-2-phosphate magnesium salt were uniformly dissolved in advance, were added and stirred. The resulting mixture was degassed under reduced pressure, and the resulting cosmetic composition was used as a test sample.

In Table 1, the "polyglyceryl-10 dioleate" is a polyglyceryl dioleate in which an average degree of polymerization of polyglycerol is 10 (component (A)). The "polyglyceryl-2 sesquicaprylate" is a polyglyceryl sesquicaprylate in which an average degree of polymerization of polyglycerol is 2 (component (E)). The "SALACOS 99" and "SALACOS 816T" are oily components (component (D)). "NOMCORT HK-G" is an oily gelling agent, which is a component (D). The evaluation results are shonwn in Table 1.

### (Evaluation)

The odor of each sample after being stored for 1 month at 50°C was evaluated in accordance with the following evaluation criteria.

⊚: sour odor wasn't determined.

○: sour odor was imperceptibly detected.
Δ○: a vague sour odor was detected,
Δ: a slight sour odor was detected,
×: sour odor was detected,
× ×: a strong sour odor was detected,

**[Table 1]**

| Examples 1 and 2, Comparative Examples 1 to 5 | (% by mass) | | | | | | |
|---|---|---|---|---|---|---|---|
| Table 1 | Example | | Comparative Example | | | | |
| | 1 | 2 | 1 | 2 | 3 | 4 | 5 |
| Polyglyceryl-10 dioleate *1 | 14 | 14 | 14 | - | 14 | 20 | 14 |
| Polyglyceryl-2 sesquicaprylate *2 | 6 | 6 | 6 | 6 | - | - | 6 |
| Isononyl isononanoate *3 | 8 | 8 | 8 | 8 | 8 | - | 8 |
| Cetyl ethylhexanoate *4 | 69.99 | 69.99 | 70 | 84 | 76 | - | 71 |
| Glyceryl (behenate/eicosanedioate) *5 | 1 | 1 | 1 | 1 | 1 | - | 1 |
| Water | 1 | 1 | 1 | 1 | 1 | 80 | - |
| Ascorbic acid | 0.01 | - | - | - | - | - | - |
| L-Ascorbic acid-2-phosphate magnesium salt | - | 0.01 | - | - | - | - | - |
| Total | 100 | 100 | 100 | 100 | 100 | 100 | 100 |
| Bad odor (after being stored at 50°C for 4 weeks) | ○ | ⊚ | × × | ⊚ | × × | × × | ⊚ |

| | | | | | | | |
|---|---|---|---|---|---|---|---|
| *1: hydroxyl value: 444.7 *2: degree of polymerization of polyglycerol: 2, sum of monoesters and diesters: 68%, mass ratio of monoesters and diesters: 1 *3: SALACOS 99 (product name, manufactured by The Nisshin OilliO Group, Ltd.) *4: SALACOS 816T (product name, manufactured by The Nisshin OilliO Group, Ltd.) *5: NOMCORT HK-G (product name, manufactured by The Nisshin OilliO Group, Ltd.) | | | | | | | |

It was confirmed that, in Comparative Example 5 not containing water as a component (B) but containing a polyglycerol fatty acid ester as a component (A), sour odor after being stored at 50°C for 4 weeks (stored at high temperature for a long period of time) was hardly detected, whereas in Comparative Examples 1, 3 and 4 not containing a compound as a component (C) but containing a polyglycerol fatty acid ester as a component (A) and water as a component (B), strong sour odor after being stored at 50°C for 4 weeks (stored at high temperature for a long period of time) was detected. In contrast, in Comparative Example 2 which included the same components as those in the sample of Comparative Example 1 except for a polyglycerol fatty acid ester as a component (A), sour odor after being stored at high temperature for a long period of time was not detected.

In contrast, in each sample of Examples 1 and 2 containing a compound as a component (C) in addition to the same components as those in the sample of Comparative Example 1, sour odor after being stored at high temperature for a long period of time was not detected. From these results, it was confirmed that, by adding the specific compound such as ascorbic acid to a cosmetic composition containing a polyglycerol fatty acid ester and water, the generation of sour odor over time was suppressed.

### [Examples 3 to 6, Comparative Example 6]

### (Production method)

All components (except for water, citric acid, sodium citrate, trehalose and sucrose) were weighed in accordance with the composition shown in Table 2, added to a beaker and mixed, and heated to 80°C to be dissolved, followed by gradually cooling while stirring. After cooling, to the obtained mixture, a mixture in which water, citric acid, sodium citrate, trehalose and sucrose were uniformly dissolved in advance, were added and stirred. The resulting mixture was degassed under reduced pressure, and the resulting cosmetic composition was used as a test sample.

### (Evaluation)

Each example was stored for 1 month at 50°C , and then the odor of each sample was evaluated every other week in accordance with the same evaluation criteria as in Example 1. The evaluation results are shown in Table 2.

**[Table 2]**

| Examples 3 to 5 | (% by mass) | | |
|---|---|---|---|
| Table 2 | Example | | |
| | 3 | 4 | 5 |
| Polyglyceryl-10 dioleate *1 | 14 | 14 | 14 |
| Polyglyceryl-2 sesquicaprylate *2 | 6 | 6 | 6 |
| Isononyl isononanoate *3 | 8 | 8 | 8 |
| Cetyl ethylhexanoate *4 | 69.99 | 69.99 | 69.99 |
| Glyceryl (behenate/eicosanedioate) *5 | 1 | 1 | 1 |
| Water | 1 | 1 | 1 |
| Citric acid | 0.02 | - | - |
| Sodium citrate | 0.08 | - | - |
| Trehalose | - | 0.01 | - |
| Sucrose | - | - | 0.01 |
| Total | 100 | 100 | 100 |
| Bad odor (after being stored at 50°C for 1 weeks) | Δ | ○ | ○ |
| Bad odor (after being stored at 50°C for 2 weeks) | Δ | ○ | ○ |
| Bad odor (after being stored at 50°C for 3 weeks) | Δ | ○ | Δ |
| Bad odor (after being stored at 50°C for 4 weeks) | Δ | Δ | Δ○ |

| | | | |
|---|---|---|---|
| *1: hydroxyl value: 447.7 *2: degree of polymerization of polyglycerol: 2, sum of monoesters and diesters: 68%, mass ratio of monoesters and diesters: 1 *3: SALACOS 99 (product name, manufactured by The Nisshin OilliO Group, Ltd.) *4: SALACOS 816T (product name, manufactured by The Nisshin OilliO Group, Ltd.) *5: NOMCORT HK-G (product name, manufactured by The Nisshin OilliO Group, Ltd.) | | | |

It was confirmed that, in each sample of Examples 3 to 5 including a compound as a component (C), although there were differences in the degree of effect, generation of sour odor after being storage at high temperature was suppressed, as compared to the sample of Comparative Example 1.

### [Comparative Examples 6 and 7]

### (Production method)

All components except for water were weighed in accordance with the composition shown in Table 3, added to a beaker and mixed, and heated to 80°C to be dissolved, followed gradually cooling while stirring. After cooling, to the obtained mixture, water was added and stirred. The resulting mixture was degassed under reduced pressure, and the resulting cosmetic composition was used as a test sample.

### (Evaluation)

Each example was stored for 1 month at 50°C, and then the odor of each sample was evaluated in accordance with the same evaluation criteria as in Example 1. The evaluation results are shown in Table 3.

**[Table 3]**

| Comparative Examples 6 and 7 | (% by mass) | |
|---|---|---|
| Table 3 | Comparative Example | |
| | 6 | 7 |
| Polyglyceryl-10 dioleate *1 | 14 | 14 |
| Polyglyceryl-2 sesquicaprylate *2 | 6 | 6 |
| Isononyl isononanoate *3 | 8 | 8 |
| Cetyl ethylhexanoate *4 | 69.95 | 69.9 |
| Glyceryl (behenate/eicosanedioate) *5 | 1 | 1 |
| Water | 1 | 1 |
| Tocopherol *6 | 0.05 | - |
| BHT *7 | - | 0.1 |
| Total | 100 | 100 |
| Bad odor (after being stored at 50°C for 4 weeks) | × | × |

| | | |
|---|---|---|
| *1: hydroxyl value: 447.7 *2: degree of polymerization of polyglycerol: 2, sum of monoesters and diesters: 68%, mass ratio of monoesters and diesters: 1 *3: SALACOS 99 (product name, manufactured by The Nisshin OilliO Group, Ltd.) *4: SALACOS 816T (product name, manufactured by The Nisshin OilliO Group, Ltd.) *5: NOMCORT HK-G (product name, manufactured by The Nisshin OilliO Group, Ltd.) *6: TOCOPHEROL 100 (product name, manufactured by The Nisshin OilliO Group, Ltd.) *7: YOSHINOX BHT (product name, manufactured by Yoshitomi Fine Chemicals Ltd.) | | |

Tocopherol and BHT (2,6-di-t-butyl-p-cresol) exhibit antioxidant effect. In each sample of Comparative Example 6 and 7 where these compounds were added instead of the component (C), sour odor was detected after being stored for 4 weeks at 50°C, like as Comparative Example 1 not containing a compound as a component (C). It was confirmed that generation of sour odor from glycerol fatty acid ester over time wasn't suppressed by the aforementioned antioxidants. From the results, it was shown that the deodorant effect of ascorbic acid or a salt of derivatives thereof was not derived from the antioxidant effect of them.

### [Examples 6 to 13]

### (Production method)

All components (except for water, ascorbic acid, L-ascorbic acid-2-phosphate magnesium salt and sodium citrate) were weighed in accordance with the composition shown in Table 4, added to a beaker and mixed, and heated to 80°C to be dissolved, followed by gradually cooling while stirring. After cooling, to the obtained mixture, a mixture in which water, ascorbic acid, L-ascorbic acid-2-phosphate magnesium salt and sodium citrate were uniformly dissolved in advance, were added and stirred. The resulting mixture was degassed under reduced pressure, and the resulting cosmetic composition was used as a test sample.

### (Evaluation)

### - Odor

Each example was stored for 1 month at 50°C, and then the odor of each sample was evaluated in accordance with the same evaluation criteria as in Example 1. The evaluation results are shown in Table 4.

### - Cleansing power

A lipstick was applied to a forearm within the area of 2cm × 2cm, and massage was conducted with 0.5 g of each composition for 30 seconds so as to remove the dirt due to lipstick, followed by washing with water. The degree of cleaned dirt (trace of lipstick) was visually observed, and evaluated in accordance with the following evaluation criteria. The evaluation results are shown in Table 4.
⊚: lipstick was completely washed out.
○: lipstick was almost washed out.
×: lipstick was not completely washed out.

**[Table 4] Examples 8, 9, 12 are reference examples.**

| | Example | | | | | | | |
|---|---|---|---|---|---|---|---|---|
| | 6 | 7 | 8 | 9 | 10 | 11 | 12 | 13 |
| Polyglyceryl-10 dioleate *1 | 14 | 14 | 14 | 14 | 14 | 14 | 14 | 14 |
| Polyglyceryl-2 sesquicaprylate *2 | 6 | 6 | 6 | 6 | 6 | 6 | 6 | 6 |
| Isononyl isononanoate | 8 | 8 | 8 | 8 | 8 | 8 | 8 | 8 |
| Cetyl ethylhexanoate | 60 | 60.9 | 50 | 20 | 69.84 | 69.99 | 10 | 69.999 |
| Glyceryl (behenate/eicosanedioate) | 1 | 1 | 1 | 1 | 1 | 1 | 1 | 1 |
| Water | 10 | 10 | 20 | 50 | 1 | 1 | 60 | 1 |
| L-Ascorbic acid-2-phosphate magnesium salt | 1 | 0.1 | 1 | 1 | 0.1 | - | 1 | 0.001 |
| L-Ascorbic acid glucoside | - | - | - | - | - | 0.01 | - | - |
| Sodium citrate | - | - | - | - | 0.06 | - | - | - |
| Total | 100 | 100 | 100 | 100 | 100 | 100 | 100 | 100 |
| Bad odor (after being stored at 50°C for 4 weeks) | ○ | ○ | Δ○ | Δ○ | Δ○ | ○ | Δ○ | ○ |
| Cleansing power | ⊚ | ⊚ | ○ | ○ | ⊚ | ⊚ | × | ⊚ |

| | | | | | | | | |
|---|---|---|---|---|---|---|---|---|
| *1: hydroxyl value: 447.7 *2: degree of polymerization of polyglycerol: 2, sum of monoesters and diesters: 68%, mass ratio of monoesters and diesters: 1 | | | | | | | | |

It was confirmed that, in the each cosmetic composition of Examples 6 to 13 containing components (A), (B) and (C), generation of sour odor after being stored at high temperature was suppressed and most of the compositions had excellent cleansing power.

### [Examples 14 to 18, Comparative Example 8]

### (Production method)

Water, L-ascorbic acid-2-phosphate magnesium salt and sodium citrate were uniformly dissolved in accordance with the composition shown in Table 5. Then, the mixture was added to polyglyceryl dioleate-10 and stirred. The resulting mixture was degassed under reduced pressure, and the resulting cosmetic composition was used as a test sample.

### (Evaluation)

### - Odor

Each example was stored for 1 month at 50°C, and then the odor of each sample was evaluated in accordance with the same evaluation criteria as in Example 1. The evaluation results are shown in Table 5.

**[Table 5] Examples 14 to 18 are reference examples.**

| | Example | | | | | Comparative Example |
|---|---|---|---|---|---|---|
| | 14 | 15 | 16 | 17 | 18 | 8 |
| Polyglyceryl-10 dioleate *1 | 50 | 50 | 50 | 50 | 50 | 50 |
| Water | 43 | 49 | 49.9 | 49 | 40 | 50 |
| L-Ascorbic acid-2-phosphate magnesium salt | 7 | 1 | - | - | - | - |
| Sodium citrate | - | - | 0.1 | 1 | 10 | 0 |
| Total | 100 | 100 | 100 | 100 | 100 | 100 |
| Bad odor (after stored at 50°C for 4 weeks) | Δ○ | ○ | Δ○ | ○ | Δ | ×× |

| | | | | | | |
|---|---|---|---|---|---|---|
| *1: hydroxyl value: 447.7 | | | | | | |

It was confirmed that, in Examples 14 to 18, generation of sour odor after being stored at high temperature was suppressed, as compared to Comparative Example 8 not including a component (C).

### [Example 19, Comparative Example 9]

### (Production method)

All components (except for water, sodium citrate, propylene glycol and glycerol) were weighed in accordance with the composition shown in Table 6, added to a beaker and mixed, and heated to 80°C to be dissolved, followed by gradually cooling while stirring. After cooling, to the obtained mixture, a mixture in which water, sodium citrate, propylene glycol and glycerol were uniformly dissolved in advance, were added and stirred. The resulting mixture was degassed under reduced pressure, and the resulting cosmetic composition was used as a test sample.

### (Evaluation)

### - Odor

Each example was stored for 1 month at 50°C, and then the odor of each sample after being stored for 1 month at 50°C was evaluated in accordance with the same evaluation criteria as in Example 1. The evaluation results are shown in Table 6.

**[Table 6]**

| | Example | Comparative Example |
|---|---|---|
| | 19 | 9 |
| Polyglyceryl-10 oleate *1 | 4.25 | 4.25 |
| Polyglyceryl-2 oleate *2 | 0.75 | 0.75 |
| Liquid paraffin | 40 | 40 |
| Triethylhexanoin *3 | 37.5 | 37.5 |
| Hydrogenated lecithin *4 | 1.5 | 1.5 |
| Water | 3.59 | 3.6 |
| Sodium citrate | 0.01 | 0 |
| Propylene glycol | 1 | 1 |
| Glycerine | 11.4 | 11.4 |
| Total | 100 | 100 |
| Bad odor (after being stored at 50°C for 4 weeks) | ○ | ×× |

| | | |
|---|---|---|
| *1: hydroxyl value: 604.4 *2: degree of polymerization of polyglycerol: 2, sum of monoesters and diesters: 100%, mass ratio of monoesters and diesters: 11 *3: T.I.O (product name, manufactured by The Nisshin OilliO Group, Ltd.) *4: BASIS LS-60HR (product name, manufactured by The Nisshin OilliO Group, Ltd.) | | |

It was confirmed that, in Example 19, generation of sour odor after being stored at high temperature was suppressed, as compared to Comparative Example 9 not containing a component (C).

### INDUSTRIAL APPLICABILITY

According to the present invention, there is provided a cosmetic composition including a polyglycerol fatty acid ester, which suppresses generation of bad odor over time in spite of the presence of water and which exhibits excellent cleansing power, and a cosmetic including the cosmetic composition.

## Claims

1. A cosmetic composition comprising a component (A), a component (B), a component (C), and a component (D), wherein
the component (A) is a polyglycerol fatty acid ester, which is an ester of polyglycerol having an average degree of polymerization of 3 to less than 100 with a fatty acid having 6 to 30 carbon atoms,
the component (B) is water,
the component (C) is at least one water-soluble compound selected from the group consisting of ascorbic acid, salts of ascorbic acid, ascorbic acid derivatives, salts of ascorbic acid derivatives, trehalose, sucrose, citric acid, and salts of citric acid, and
the component (D) is at least one oil component selected from the group consisting of natural animal and vegetable fats and oils, semi-synthetic oils, hydrocarbon oils, higher fatty acids, synthetic ester oils, silicone oils, essential oil constituents of animal and vegetable fats and oils, fat-soluble vitamins, phospholipids, higher alcohols, and fluorine-containing oils, wherein
the ascorbic acid derivatives is at least one selected from the group consisting of L-ascorbic acid glucoside, L-ascorbic acid-2-phosphate, L-ascorbic acid-3-phosphate, L-ascorbic acid-6-phosphate, L-ascorbic acid-2-polyphosphate, and L-ascorbic acid-2-sulfate, and wherein
the amount of the component (A) in the cosmetic composition is 1 to 80% by mass relative to the total amount of the cosmetic composition, the amount of the component (B) in the cosmetic composition is 0.001 to 10% by mass relative to the total amount of the cosmetic composition,
the amount of the component (C) in the cosmetic composition is 0.005 to 20% by mass relative to the amount of component (A),
the amount of the component (D) in the cosmetic composition is 10 to 90% by mass relative to the total amount of the cosmetic composition.

2. The cosmetic composition according to Claim 1, further comprising a component (E), wherein
the component (E) is a non-ionic surfactant, provided that the component (A) is excluded from the component (E).

3. The cosmetic composition according to Claim 2, wherein
the non-ionic surfactant as a component (E) is a polyhydric alcohol fatty acid ester, which is an ester of a fatty acid having 6 to 22 carbon atoms with a polyhydric alcohol having 2 to 4 hydroxyl groups, wherein
the sum of monoesters and diesters is at least 50% by mass relative to the total amount of the polyhydric alcohol fatty acid ester, and the mass ratio of monoesters and diesters represented by [mass of monoesters] / [mass of diesters] is 12 or less.

4. The cosmetic composition according to Claim 3, wherein
the polyhydric alcohol is at least one member selected from the group consisting of glycerin, diglycerin, sorbitan, and pentaerythritol.

5. Use of the cosmetic composition according to any one of Claims 1 to 4, for a raw material of a cleansing cosmetic or a bath cosmetic.

## Patentansprüche

1. Eine kosmetische Zusammensetzung, umfassend eine Komponente (A), eine Komponente (B), eine Komponente (C) und eine Komponente (D), wobei die Komponente (A) ein Fettsäure-Polyglycerinester ist, bei dem es sich um einen Polyglycerinester mit einem mittleren Polymerisationsgrad von 3 bis weniger als 100 handelt, mit einer Fettsäure die zwischen 6 und 30 Kohlenstoffatome hat,
die Komponente (B) ist Wasser,
die Komponente (C) ist mindestens eine wasserlösliche Verbindung, ausgewählt aus der Gruppe bestehend aus Ascorbinsäure, Salzen der Ascorbinsäure, Derivaten der Ascorbinsäure, Trehalose, Sucrose, Zitronensäure und Salzen der Zitronensäure und die Komponente (D) ist mindestens eine Ölkomponente, ausgewählt aus der Gruppe bestehend aus natürlichen tierischen und pflanzlichen Fetten und Ölen, halbsynthetischen Ölen, Kohlenwasserstoffölen, höheren Fettsäuren, synthetischen Esterölen, Silikon- Öl, essentiellen Ölbestandteilen tierischer und pflanzlicher Fette und Öle, fettlöslichen Vitaminen, Phospholipiden, höheren Alkoholen und Fluorverbindungen enthaltenden Ölen, wobei
bei den Ascorbinsäurederivaten wird mindestens eine ausgewählt aus der Gruppe bestehend aus L-Ascorbinsäure-Glucosid, L-Ascorbinsäure-2-phosphat, L-Ascorbinsäure-3-phosphat, L-Ascorbinsäure-6-phosphat, L-Ascorbinsäure-2-polyphosphat und L-Ascorbinsäure-2-sulfat, und wobei
der Anteil der Komponente (A) in der kosmetischen Zusammensetzung 1 bis 80 Masse-% bezogen auf die Gesamtmenge der kosmetischen Zusammensetzung beträgt, der Anteil der Komponente (B) in der kosmetischen Zusammensetzung 0,001 bis 10 Masse-% bezogen auf die Gesamtmenge der kosmetischen Zusammensetzung beträgt,
der Anteil der Komponente (C) in der kosmetischen Zusammensetzung 0,005 bis 20 Masse-% bezogen auf die Menge der Komponente (A) beträgt,
der Anteil der Komponente (D) in der kosmetischen Zusammensetzung 10 bis 90 Masse-% bezogen auf die Gesamtmenge der kosmetischen Zusammensetzung beträgt.

2. Die kosmetische Zusammensetzung nach Anspruch 1, weiterhin eine Komponente (E) umfassend, wobei
die Komponente (E) ein nicht-ionisches Tensid ist, vorausgesetzt die Komponente (A) ist aus der Komponente (E) ausgeschlossen.

3. Die kosmetische Zusammensetzung nach Anspruch 2, wobei
das nicht-ionische Tensid als Komponente (E) ist ein mehrwertiger Alkohol-Fettsäureester, bei dem es sich um einen Ester einer Fettsäure mit 6 bis 22 Kohlenstoffatomen handelt, mit einem mehrwertigen Alkohol mit 2 bis 4 HydroxylGruppen, worin
die Summe von Monoestern und Diestern mindestens 50 Masse-%, bezogen auf die Gesamtmenge des mehrwertigen Alkohol-Fettsäureester beträgt und das Masserverhältnis von Monoestern und Diestern gebildet durch [Masse der Monoester] / [Masse der Diester] 12 oder weniger beträgt.

4. Die kosmetische Zusammensetzung nach Anspruch 3, wobei
es sich bei dem mehrwertigen Alkohol um mindestens einen aus der Gruppe bestehend aus Glyzerin, Diglyzerin, Sorbitan und Pentaerythritol handelt.

5. Verwendung der kosmetischen Zusammensetzung nach einem der Ansprüche 1 bis 4 als Rohmaterial für Reinigungs- oder Badekosmetika

## Revendications

1. Composition cosmétique comprenant un composant (A), un composant (B), un composant (C) et un composant (D), dans laquelle
le composant (A) est un ester d'acide gras de polyglycérol, qui est un ester de polyglycérol ayant un degré moyen de polymérisation de 3 à moins de 100 avec un acide gras comportant de 6 à 30 atomes de carbone,
le composant (B) est de l'eau,
le composant (C) est au moins un composé hydrosoluble choisi dans le groupe constitué de l'acide ascorbique, des sels de l'acide ascorbique, des dérivés de l'acide ascorbique, des sels de dérivés de l'acide ascorbique, du tréhalose, du sucrose, de l'acide citrique et des sels de l'acide citrique, et
le composant (D) est au moins un composant huileux choisi dans le groupe constitué des graisses et huiles animales et végétales naturelles, des huiles semi-synthétiques, des huiles d'hydrocarbures, des acides gras supérieurs, des huiles d'ester synthétiques, des huiles de silicone, des constituants d'huiles essentielles de graisses et huiles animales et végétales, des vitamines liposolubles, des phospholipides, des alcools supérieurs et des huiles contenant du fluor, dans laquelle les dérivés de l'acide ascorbique sont au moins un choisi dans le groupe constitué du glucoside d'acide L-ascorbique, de l'acide L-ascorbique-2-phosphate, de l'acide L-ascorbique-3-phosphate, de l'acide L-ascorbique-6-phosphate, de l'acide L-ascorbique-2-polyphosphate et de l'acide L-ascorbique-2-sulfate, et dans laquelle la quantité du composant (A) dans la composition cosmétique est de 1 à 80 % en masse par rapport à la quantité totale de la composition cosmétique, la quantité du composant (B) dans la composition cosmétique est de 0,001 à 10 % en masse par rapport à la quantité totale de la composition cosmétique,
la quantité du composant (C) dans la composition cosmétique est de 0,005 à 20 % en masse par rapport à la quantité de composant (A),
la quantité du composant (D) dans la composition cosmétique est de 10 à 90 % en masse par rapport à la quantité totale de la composition cosmétique.

2. Composition cosmétique selon la revendication 1, comprenant en outre un composant (E), dans laquelle
le composant (E) est un tensioactif non ionique, à condition que le composant (A) soit exclu du composant (E).

3. Composition cosmétique selon la Revendication 2, dans laquelle
le tensioactif non ionique en tant que composant (E) est un ester d'acide gras d'alcool polyhydrique, qui est un ester d'un acide gras comportant de 6 à 22 atomes de carbone avec un alcool polyhydrique comportant de 2 à 4 groupes hydroxyles, dans laquelle
la somme des monoesters et des diesters est d'au moins 50 % en masse par rapport à la quantité totale de l'ester d'acide gras d'alcool polyhydrique, et le rapport massique des monoesters et des diesters représenté par [masse des monoesters]/[masse des diesters] est de 12 ou moins.

4. Composition cosmétique selon la Revendication 3, dans laquelle
l'alcool polyhydrique est au moins un élément choisi dans le groupe constitué de la glycérine, de la diglycérine, du sorbitan et du pentaérythritol.

5. Utilisation de la composition cosmétique selon l'une quelconque des Revendications 1 à 4, pour une matière première d'un cosmétique nettoyant ou d'un cosmétique pour le bain.
